# EUROPEAN PATENT APPLICATION

(11) **EP 4 167 247 A1**
(43) Date of publication of application: **19.04.2023**
(21) Application number: 20941724.5
(22) Date of filing: 24.09.2020
(51) Int. Cl.: G16H 50/30, G16H 50/20, G16H 10/60, G16H 10/20, G16H 80/00

(54) **MANAGEMENT SYSTEM FOR PROVIDING BEHAVIORAL HABIT IMPROVEMENT**

(30) Priority: 26.06.2020 KR 20200078492
(71) Applicant: Olive Healthcare Inc., Seoul 05836 (KR)
(72) Inventor: HAN, Sung Ho, Seoul 04732 (KR); KIM, Tae Min, Seoul 06249 (KR); LEE, A Lan, Seoul 05373 (KR); SHIN, A Ram, Suwon-si Gyeonggi-do 16509 (KR); GWON, Ji Young, Seoul 05259 (KR)
(74) Representative: Manitz Finsterwald Patent- und Rechtsanwaltspartnerschaft mbB
(86) International application number: PCT/KR2020/012931
(87) International publication number: WO 2021/261662

(57) **Abstract**

A management server for providing behavioral habit improvement includes a communication module, a memory configured to store a behavioral habit improvement service providing program, and a processor configured to execute the program, wherein the processor executes the program to provide a user terminal with a user interface that allows an input of behavior pattern information including behavioral habits, a trigger, and a solution, and period information for implementing the behavior pattern information, to match the behavior pattern information, the period information, and user identification information input from the user terminal, to store the matched information in a database, to generate a behavior pattern feed that displays the behavioral habits, the trigger, and the solution on one screen, and to provide the behavior pattern feed through the user interface.

## Description

### BACKGROUND

### 1. Field

The present disclosure relates to a management system for providing behavioral habit improvement.

### 2. Description of the Related Art

Recently, with the development of communication networks and user devices, the user devices (smartphones, wearable devices, or so on) are often used to manage habits in fields, such as exercise, learning, and treatment.

For example, when a habit management device is used for chronic disease treatment, the types of bad lifestyles of chronic disease patients are defined to be used in the chronic disease treatment in the form of a checklist.

In addition, when the habit management device is used for schedule and goal management, a user is helped to easily input and store his/her schedule through a user device and to check at any time.

However, since the known habit management device generates and manages a task, a schedule, and a goal as separate items, systematic management may not be performed, and the frequency of use is low due to inconvenience of individual input and search, and accordingly, management may be eventually abandoned. In addition, the known habit management device simply input and store only contents of a schedule and a goal and do not provide an analysis result according to the current progress and a progress result. Therefore, there is a problem in that it is impossible to check at which stage the current goal reaches and how to proceed.

In this regard, Korean Patent Publication No. 10-2015-0010255 (Title of Invention: APPARATUS FOR DIAGNOSTIC USING HABIT AND MATHOD FOR DIAGNOSTIC OF THEREOF) discloses a method for diagnosing a disease based on a habit generated by collecting behaviors of users and analyzing the collected behaviors.

### SUMMARY

In order to solve the problems described above, a management system for providing behavioral habit improvement according to the present disclosure matches a trigger that continuously causes bad behavioral habits to a solution that may prevent the bad behavioral habits by replacing the trigger for each behavioral habit and stores the matched result to a database. In addition, the management system presents many triggers and various solutions for the behavioral habits that need improvement based on the database, and provides a behavior pattern feed that displays behavior pattern information (behavioral habits, a trigger, and a solution) selected by a user on one screen and an implementation rate for the behavior pattern information according to the passage of time.

Technical objects to be achieved by the present embodiment are not limited to the technical objects described above, and there may be other technical objects.

According to an aspect of the present disclosure, a management server for providing behavioral habit improvement includes a communication module, a memory configured to store a behavioral habit improvement service providing program, and a processor configured to execute the behavioral habit improvement service providing program, wherein the processor executes the behavioral habit improvement service providing program to provide a user terminal with a user interface that allows an input of behavior pattern information including behavioral habits, a trigger, and a solution, and period information for implementing the behavior pattern information, to match the behavior pattern information, the period information, and user identification information input from the user terminal, to store the matched information in a database, to generate a behavior pattern feed that displays the behavioral habits, the trigger, and the solution on one screen, and to provide the behavior pattern feed through the user interface.

The processor may receive information on whether or not the behavior pattern information is implemented for each preset period from the user terminal and provide a visual image of an implementation rate based on the information on whether or not the behavior pattern information is implemented through the user interface.

The behavioral habits may correspond to a category that collectively refer to bad behavioral habits that need improvement and include lack of sleep, an eating habit problem, a drinking problem, a non-smoking problem, a bad relationship, and lack of concentration.

The behavior pattern information may be stored in the database by being composed of a hierarchical structure including a plurality of triggers that cause at least one behavioral habit in the category and a plurality of solutions for preventing the behavioral habits by replacing at least one trigger.

The processor may provide the user interface when receiving a behavior pattern information registration request from the user terminal, and the user interface may include a first function for inputting at least one behavioral habit in the category, a second function for inputting at least one trigger that causes the behavioral habits, a third function for inputting at least one solution that prevents the behavioral habits by replacing the trigger, and a fourth function for inputting the period information including a period during which the behavior pattern information is implemented, a start date, and a report date.

The processor may match, as one group, users having one or more pieces of the behavior pattern information matching each other which include the behavioral habits, the trigger, and the solution.

According to another aspect of the present disclosure, a method of providing behavioral habit improvement includes (a) providing a user terminal with a user interface that allows an input of behavior pattern information including behavioral habits, a trigger, and a solution, and period information for implementing the behavior pattern information, (b) matching the behavior pattern information, the period information, and user identification information, which are input from the user terminal, to each other and storing the matched information in a database, and(c) generating a behavior pattern feed that displays the behavioral habits, the trigger, and the solution on one screen and providing the behavior pattern feed through the user interface.

The method may further include receiving information on whether or not the behavior pattern information is implemented for each preset period from the user terminal, and providing a visual image of an implementation rate based on the information on whether or not the behavior pattern information is implemented, through the user interface.

The behavioral habits may correspond to a category that collectively refer to bad behavioral habits that need improvement and include lack of sleep, an eating habit problem, a drinking problem, a non-smoking problem, a bad relationship, and lack of concentration.

The behavior pattern information may be stored in the database by being composed of a hierarchical structure including a plurality of triggers that cause at least one behavioral habit in the category and a plurality of solutions for preventing the behavioral habits by replacing at least one trigger.

In (a), the user interface may be provided when receiving a behavior pattern information registration request from the user terminal, and the user interface may include a first function for inputting at least one behavioral habit in the category, a second function for inputting at least one trigger that causes the behavioral habits, a third function for inputting at least one solution that prevents the behavioral habits by replacing the trigger, and a fourth function for inputting the period information including a period during which the behavior pattern information is implemented, a start date, and a report date.

The present invention solves the known problems described above and matches behavior pattern information including a user's behavioral habits, a trigger, and a solution and stores the matched information in a database, and accordingly, users can share the trigger and the solution for the same behavioral habits.

In addition, the present invention receives information on whether or not the behavior pattern information is implemented during a period set by a user and provides an implementation rate of the behavior pattern information as a visual image, based on the information on whether or not the behavior pattern information is implemented, and thus, a current progress of the behavior pattern information can be checked in real time, and an analysis result according to the information on whether the behavior pattern information is implemented can be provided.

### BRIEF DESCRIPTION OF THE DRAWINGS

FIG. 1 is a configuration diagram of a management system for providing behavioral habit improvement, according to an embodiment of the present disclosure;
FIG. 2 is examples of a user interface provided upon registration of behavior pattern information, according to an embodiment of the present disclosure;
FIG. 3 is an example of a user interface provided upon registration of behavior pattern information, according to another embodiment of the present disclosure;
FIG. 4 is an example of a user interface provided upon completion of registration of behavior pattern information, according to an embodiment of the present disclosure;
FIG. 5 is examples of a user interface displaying a behavior pattern feed for displaying behavioral habits, triggers, and solutions on one screen, according to an embodiment of the present disclosure;
FIG. 6 is an example of a user interface that provides a plurality of behavior pattern feeds and an implementation period of user behavior pattern information, according to an embodiment of the present disclosure;
FIG. 7 is examples of a user interface that provides a visual image of an implementation rate based on a user's information on whether or not the behavior pattern information is implemented, according to an embodiment of the present disclosure;
FIG. 8 is examples of a user interface that provides a group list to a user by grouping users having one or more pieces of behavior pattern information matching each other into one group, according to an embodiment of the present disclosure; and
FIG. 9 is a flowchart illustrating a management method for providing behavioral habit improvement, according to an embodiment of the present disclosure.

### DETAILED DESCRIPTION

Hereinafter, embodiments of the present disclosure will be described in detail with reference to the accompanying drawings such that those skilled in the art to which the present disclosure belongs may easily implement the present disclosure. However, the present disclosure may be embodied in various different forms and is not limited to the embodiments described herein. In addition, in order to clearly illustrate the present disclosure in the drawings, parts irrelevant to the descriptions are omitted, and similar reference numerals are attached to similar parts throughout the specification.

Throughout the specification, when a portion is "connected" or "coupled" to another portion, this includes not only a case of being "directly connected or coupled" but also a case of being "electrically connected" with another element interposed therebetween. In addition, when a portion "includes" a certain component, this means that other components may be further included therein rather than excluding other components, unless otherwise stated.

In the present specification, a "portion" includes a unit implemented by hardware or software and a unit implemented by using both, and one unit may be implemented by using two or more pieces of hardware, and two or more units may be implemented by one piece of hardware. Meanwhile, "∼ portion" is not limited to software or hardware, and "∼ portion" may be configured to be in an addressable storage medium or may be configured to regenerate one or more processors. Accordingly, as an example, "∼ portion" indicates configuration elements such as software configuration elements, object-oriented software configuration elements, class configuration elements, and task configuration elements, and progresses, functions, properties, and procedures., subroutines, segments of program code, drivers, firmware, microcode, circuitry, data, databases, data structures, tables, arrays, and variables. The functions provided in the configuration elements and "- portions" may be combined into a smaller number of configuration elements and "- portions" or further separated into additional configuration elements and "- portions". In addition, configuration elements and "- portions" may be implemented to regenerate one or more CPUs in a device or secure multimedia card.

A "network" indicates a connection structure in which information may be exchanged between respective nodes such as terminals and servers and includes a local area network (LAN), a wide area network (WAN), the Internet (world wide web (WEB)), a wired and wireless data network, a telephone network, a wired and wireless television network, and so on. The wireless data communication network includes, for example, 3 generation (3G), 4G, 5G, 3rd generation partnership project (3GPP), long term evolution (LTE), world interoperability for microwave access (WIMAX), Wi-Fi, Bluetooth communication, infrared communication, ultrasonic Communication, visible light communication (VLC), LiFi, and so on but are not limited thereto.

In the present specification, a user terminal may be implemented as a computer or a mobile terminal capable of accessing a management server that improves behavioral habits through a network. Here, the computer may include, for example, a notebook computer, a desktop computer, a laptop, and so on in which a web browser is installed, and the mobile terminal may include, for example, a wireless communication device that ensures portability, that is, all kinds of handheld-based wireless communication devices such as various smartphones and tablet PCs.

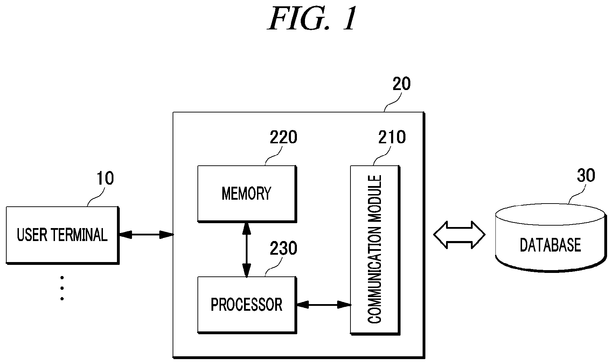

FIG. 1 is a configuration diagram of a management system for providing behavioral habit improvement, according to an embodiment of the present disclosure.

Referring to FIG. 1, the management system for providing behavioral habit improvement, according to the present disclosure, includes a plurality of user terminals 10, a management server 20 for providing behavioral habit improvement, and a database 30.

The management server 20 for providing behavioral habit improvement processes operations for providing a behavioral habit improvement service in association with a behavioral habit improvement service providing program which is executed by the user terminal 10.

For example, the user terminal 10 may include a communication module, a memory, and a processor, and the communication module of each terminal may perform data communication with the server 20 under control by the processor.

As illustrated in FIG. 1, the management server 20 includes a communication module 210, a memory 220, a processor 230, and a database 30.

The communication module 210 performs data communication with each of the user terminals 10. The communication module 210 provides a communication interface required to provide signals transmitted to and received from the user terminals 10 in the form of packet data in conjunction with a communication network. Here, the communication module 210 may include hardware and software necessary for transmitting and receiving a signal, such as a control signal or a data signal, through a wired/wireless connection to another network device.

The behavioral habit improvement service providing program for providing a behavioral habit improvement service to the user terminals 10 is stored in the memory 220, and the behavioral habit improvement service providing program stored in the memory may be driven by the processor 230.

In addition, the memory 220 temporarily or permanently stores the data processed by the processor 230. Here, the memory 220 may include volatile storage media or nonvolatile storage media, but the scope of the present disclosure is not limited thereto.

The memory 220 may store a separate program, such as an operating system for processing and controlling the processor 230, or may perform a function for temporarily storing input or output data.

The memory 220 may include at least one type of storage medium among a flash memory type, a hard disk type, a multimedia card micro type, a card type memory (for example, a secure digital (SD) memory, an extreme digital (XD) memory, or so on), a random access memory (RAM), and a read only memory (ROM). In addition, the user terminal 10 may also operate a web storage that performs a storage function of the memory 220 on the Internet.

The processor 230 executes the program stored in the memory 220 and performs processing corresponding to each operation of the behavior habit improvement service providing program processed by a processor of the user terminals 10 to be described below.

The processor 230 executes the behavioral habit improvement service providing program stored in the memory 220 and controls all operations for providing the behavioral habit improvement service.

To this end, the processor 230 include at least one processing unit (a central processing unit (CPU), a micro-processor, a digital signal processor (DSP), and so on), a RAM, a ROM, and so on and may read the program stored in the memory 220 into RAM to be executed by at least one processing unit. In addition, a term "processor" may be interpreted as the same meaning as terms, such as a "controller", a "computing device", and a "control unit" depending on embodiments.

A schematic procedure of providing the behavioral habit improvement service provided through the behavioral habit improvement service providing program, according to an embodiment of the present disclosure is as follows.

Subsequently, the processor 230 may provide the user terminal 10 with a user interface that allows input of behavior pattern information including behavioral habits, triggers, and solutions, and period information for implementing the behavior pattern information, match the behavior pattern information, the period information, and user identification information input from the user terminal 10 and store the matched information in the database 30, generate a behavior pattern feed for displaying the behavioral habits, the triggers, and the solutions on one screen, and provide the behavior pattern feed through a user interface.

In addition, the processor 230 may receive, from the user terminal 10, information on whether or not the behavior pattern information is implemented for each preset period, and may provide a visual image of an implementation rate based on the information on whether or not the behavior pattern information is implemented through the user interface.

Therefore, numerous triggers and various solutions for behavioral habits requiring improvement may be presented, and users may share the triggers and solutions for the same behavioral habits. In addition, it is possible to provide a behavior pattern feed that displays behavior pattern information (behavior habits, triggers, and solutions) selected by the user on one screen, and an implementation rate for the behavior pattern information according to lapse of time.

The database 30 stores data accumulated while performing a program for providing a service related to the behavioral habit improvement service providing program. For example, the database 30 may store a plurality of behavioral habits, a plurality of triggers that cause at least one behavioral habit, and a plurality of solutions for preventing the behavioral habits by replacing the triggers, which are matched to each other.

For example, the behavioral habit may correspond to a category that collectively refers to bad behavioral habits required to be improved and may include lack of sleep, an eating habit problem, a drinking problem, a non-smoking problem, a bad relationship, lack of concentration, and so on. For example, the trigger may include a trigger of "using a mobile phone before going to bed " as a cause of lack of sleep (behavioral habit). In this case, a solution therefor may include a solution of "putting the mobile phone in another room " as a solution to prevent lack of sleep (behavioral habit) by replacing the trigger ("I use my phone before going to bed"). In addition, the trigger ("I use my mobile phone before going to bed") is not only matched to one behavioral habit (lack of sleep) but may also be matched to other behavioral habits (lack of concentration and so on).

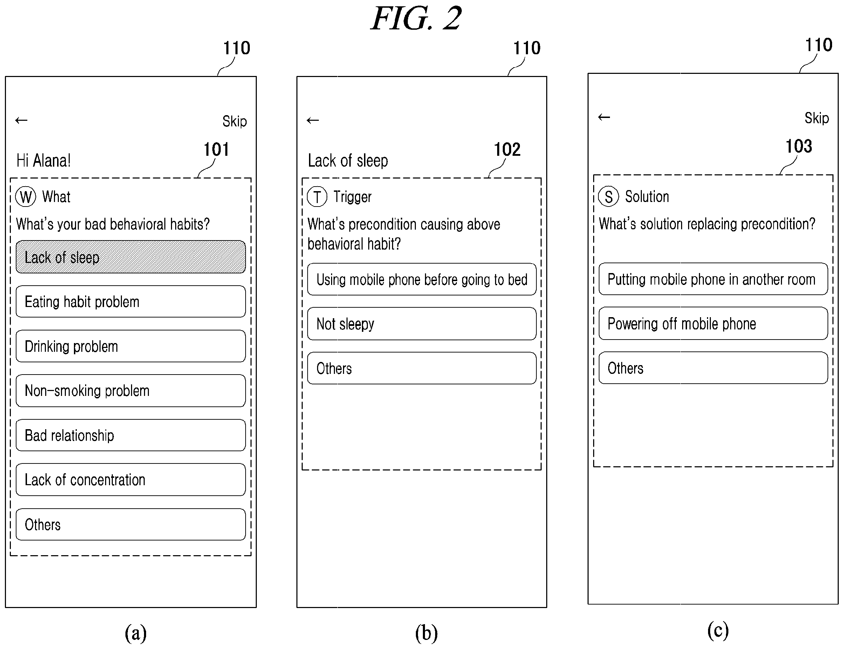

Here, a method of providing a behavioral habit improvement service performed by the processor 230 will be described in more detail with reference to FIGS. 2 to 7 below.

FIG. 2 is examples of a user interface provided upon registration of behavior pattern information, according to an embodiment of the present disclosure.

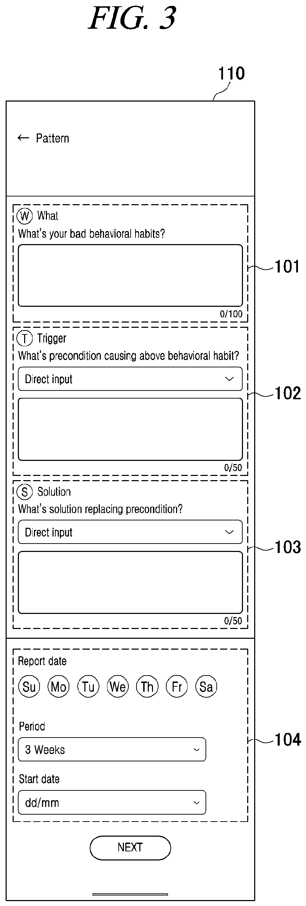

FIG. 3 is an example of a user interface provided upon registration of behavior pattern information, according to an embodiment of the present disclosure.

Referring to FIG. 2, the processor 230 may provide user interfaces 110 when receiving a behavior pattern information registration request from the user terminal 10.

For example, referring to FIG. 2(a), the user interface 110 for registering behavior pattern information includes a first function 101 of inputting at least one behavioral habit among categories.

In one example, as illustrated in FIG. 2(a), a user may select one of the categories of behavioral habits stored in the database 30 through the first function 101 of the user interface 110.

In another example, as illustrated in FIG. 3, the user may directly input a behavioral habit not stored in the database 30 through the first function 101 of the user interface 110.

Here, the behavioral habit is a category that collectively refers to bad behavioral habit required to be improved and may include lack of sleep, an eating habit problem, a drinking problem, a non-smoking problem, a bad relationship, and lack of concentration, but is not limited thereto.

In addition, the behavior pattern information may be composed of a hierarchical structure, in which a plurality of triggers that cause at least one behavioral habit among categories and a plurality of solutions for preventing a behavioral habit by replacing at least one trigger and may be stored in the database 30.

For example, the lack of sleep may match a first trigger of "using a mobile phone before going to bed", and the first trigger may match a plurality of solutions, such as a first solution of "putting the mobile phone in another room" and a second solution of "setting licking of a mobile phone during the evening".

For example, referring to FIG. 2(b), the user interface 110 may include a second function 102 of inputting at least one trigger that causes behavioral habits. In another example, as illustrated in FIG. 3, a user may directly input a trigger not stored in the database 30 through the second function 102 of the user interface 110.

In addition, referring to FIG. 2(c), the user interface 110 may include a third function 103 of inputting at least one solution for preventing behavioral habits by replacing the trigger. In another example, as illustrated in FIG. 3, a user may directly input a solution not stored in the database 30 through the third function 103 of the user interface 110.

Referring to FIG. 3, the user interface 110 may include a fourth function 104 of inputting period information including a period in which behavior pattern information is implemented, a start date, and a report date.

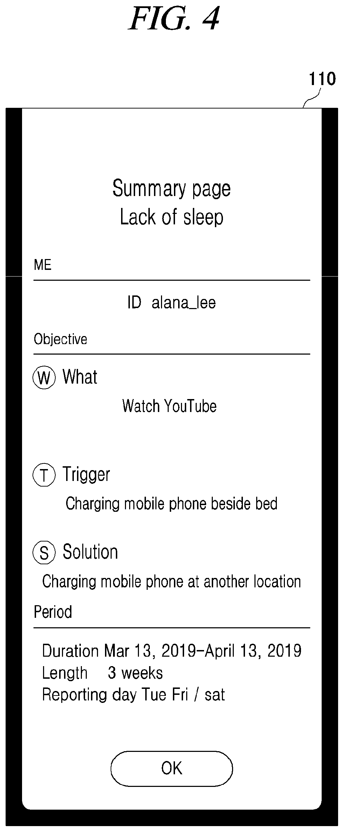

FIG. 4 is an example of a user interface provided upon completion of registration of behavior pattern information, according to an embodiment of the present disclosure.

Referring to FIG. 4, when receiving behavior pattern information and period information from the user terminal 10, the processor 230 may provide a summary page of the behavior pattern information through the user interface 110.

For example, identification information for intuitively distinguishing behavioral habits W, a trigger T, and a solution S may be displayed on a summary page, and each piece of the identification information may be displayed in letters and colors. In addition, period information including a period in which the behavior pattern information is implemented, a date, and a notification report date may be displayed. Through this summary page, a user may easily check behavioral habits that the user wants to improve at any time. In addition, since the summary page is generated for each of the behavioral habits to be improved, a user may conveniently check a target to be improved by displaying a plurality of adjacent summary pages.

That is, the processor 230 may match the behavior pattern information, the period information, and the user identification information, which are input from the user terminal 10, to each other and store the matched information in the database 30.

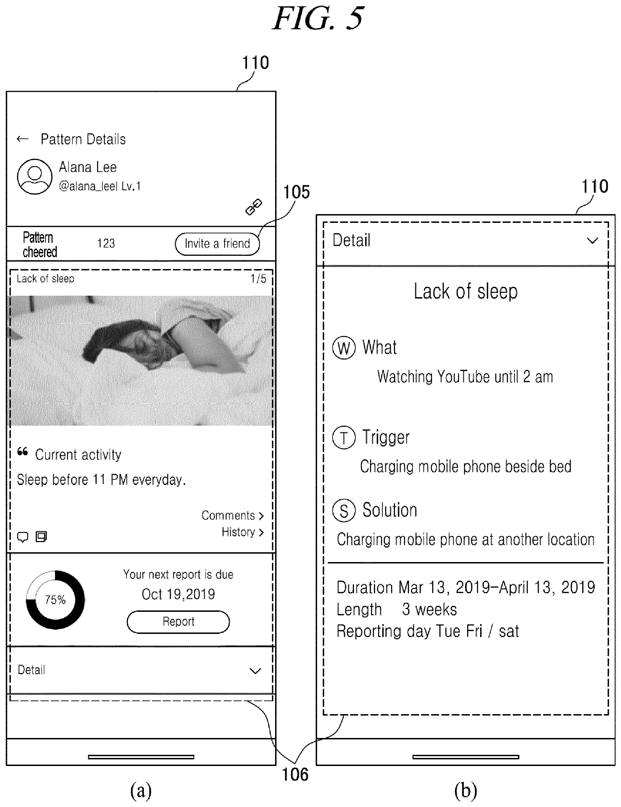

FIG. 5 is examples of a user interface displaying a behavior pattern feed for displaying behavioral habits, a trigger, and a solution on one screen, according to an embodiment of the present disclosure.

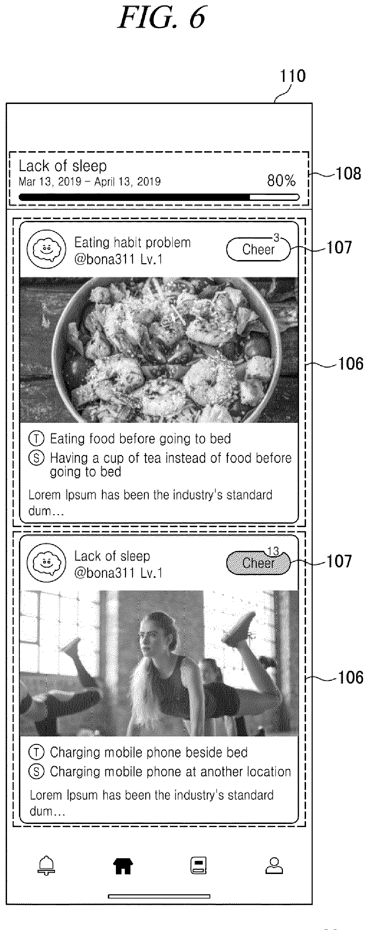

FIG. 6 is an example of a user interface providing a plurality of behavior pattern feeds and an implementation period of user behavior pattern information, according to an embodiment of the present disclosure.

Referring to FIG. 6, the processor 230 may generate a behavior pattern feed 106 that displays, on a screen, behavioral habits, triggers, and solutions which are stored based on user identification information, and provide the behavior pattern feed 106 to the user interface 110.

For example, as illustrated in FIG. 5(a), the user interface 110 may provide a friend invitation function 105 that may disclose the behavior pattern feed 106 including a user's behavior pattern information and period information to friends.

For example, as illustrated in FIG. 5(b), the behavior pattern feed 106 registered in the management system for providing behavioral habit improvement, according to the present disclosure, may provide a detail view function that displays in detail the user's behavior pattern information and period information.

For example, as illustrated in FIG. 6, the user interface 110 may provide disclosed behavior pattern feeds 106 of other users in the form of thumbnail. In this case, the thumbnail necessarily includes a trigger and a solution distinguished by identification information.

The user interface 110 may provide a cheer function 107, and a user may register a cheer mark in the disclosed behavior pattern feed 106 of another user in the form of a thumbnail through the cheer function 107. In addition, as illustrated in FIG. 5(a), a cheer message may be registered through a comment function provided on the entire view screen of the behavior pattern feed 106.

For example, when a user registers one behavioral habit of "lack of sleep", the processor 230 may provide the behavior pattern feeds 106 of other users whose behavioral habits correspond to the "lack of sleep". In another example, when a user registers two behavioral habits, "lack of sleep" and "eating habit problem", the processor 230 may provide behavior pattern feeds 106 of other users whose behavioral habits correspond to the "lack of sleep" or the "eating habit problem".

Therefore, a user may form a sense of kinship and consensus through the behavior pattern feeds 106 of other users having a behavioral habit identical to the behavioral habit input by the user and may maintain motivation for providing behavioral habit improvement through a cheer display or a cheer message for the behavior pattern feed 106.

In addition, referring to FIG. 6, the user interface 110 may provide a progress bar function 108 that provides an elapse rate according to elapse time based on period information set by a user. Through this, the user may increase his or her practical understanding of a target period in order to improve behavioral habits.

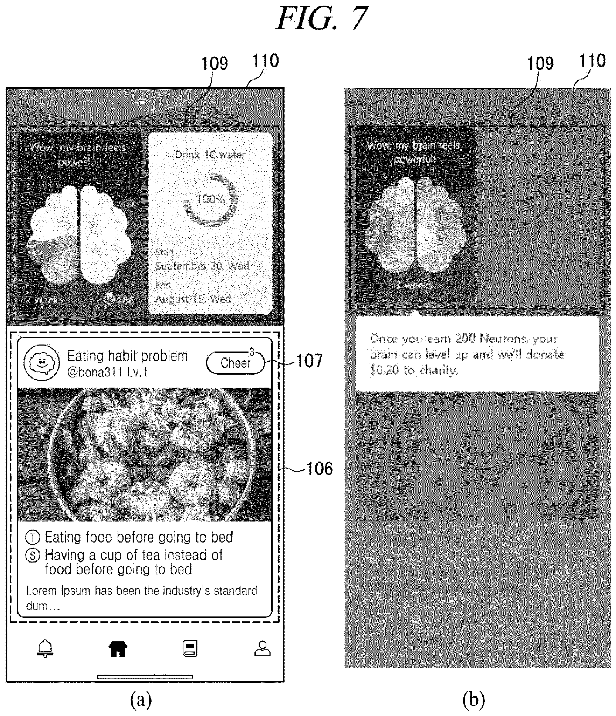

FIG. 7 is examples of a user interface that provides a visual image of an implementation rate based on a user's information on whether or not the behavior pattern information is implemented, according to an embodiment of the present disclosure.

Referring to FIG. 7, the processor 230 may provide a compensation function 109 that receives information on whether or not the behavior pattern information is implemented for each preset period from the user terminal 10 and displays a visual image of an implementation rate based on the information on whether or not the behavior pattern information is implementedthrough the user interface 110.

For example, the user terminal 10 may input, through the user interface 110, information on whether or not to implement the behavior pattern information notified in the form of a push notification. In one example, when a user registers behavior pattern information including a first trigger of "using the mobile phone before sleeping " and a first solution of "putting the mobile phone in another room" for the behavioral habit of lack of sleep (can't sleep), the user may periodically (for example, daily) record whether or not the first solution is implemented.

In addition, the processor 230 may provide a compensation function 109 that displays a visual image of an implementation rate based on the information on whether or not the behavior pattern information is implemented which is received from the user terminal 10 through the user interface 110.

For example, as illustrated in FIG. 7, the compensation function 109 may visualize a degree of activation of neurons corresponding to the implementation rate as an image and display the image. For example, when the behavior pattern information is fully implemented during a period (for example, three weeks) set by a user, a reward point may be provided along with the completed neuron image. In another example, when the behavior pattern information is not fully implemented during the period set by the user, an incomplete neuron image and a point corresponding to the implementation rate may be provided.

For example, when a first user inputs failure information (for example, an implementation rate less than 50 %) for a first solution, the processor 230 may provide a new second solution. For example, the processor 230 may suggest, as an alternative, an n-th solution (for example, an implementation rate greater than or equal to 50 %) that is successful among users (matched users) having the same registered trigger as the first user. In addition, when a new n-th solution is successful after failing with the same first solution among the matched users, the corresponding n-th solution may be recommended to the first user.

In a further embodiment, when behavioral habits other than the first behavioral habit input by a user is included among triggers or solutions matched to the first behavioral habit input by the user, the processor 230 may generate bundled behavior pattern information in which two or more behavioral habits are packaged.

For example, the first trigger of "eating before going to bed" and the first solution of "drinking a cup of tea instead of food before going to bed" may respectively be a trigger and a solution matching the second behavioral habit of "lack of sleep" in addition to the first behavioral habit of "eating habit problem".

In this case, by implementing the first solution (drinking a cup of tea before going to bed) selected by the user, behavioral patterns for improving the first behavioral habit (the eating habit problem) targeted by the user and the second behavioral habit (the lack of sleep) may be implemented.

That is, the present disclosure may visually provide that other bad behavioral habits are improved in addition to the bad behavioral habit that the user expects to improve by implementing one solution (that is, behavior pattern information), and thus, motivation to improve behavioral habits may be maintained.

. 8 is examples of a user interface for providing a group list to a user after users having one or more pieces of behavior pattern information matching each other are matched into one group, according to an embodiment of the present disclosure.

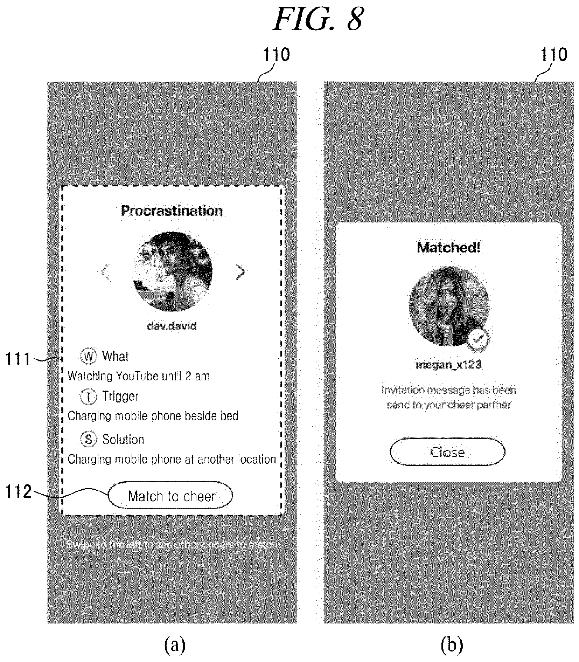

Referring to FIG. 8, the processor 230 may match, as one group, the users having one or more pieces of behavior pattern information matching each other which include behavioral habits, triggers, and solutions. That is, the user may be provided with a list of other users having one or more pieces of behavior pattern information identical to behavior pattern information of the user.

For example, the processor 230 may match users having behavioral habits identical to triggers as a first group and match users having the same period information in the first group as a 1-1-th group. The processor 230 may match users having behavioral habits identical to solutions as a second group and match users having the same period information in the second group as a 2-1-th group. In addition, in case of users who are not included in the first grout, the 1-1-th group, the second group, and the 2-1-th group, users having the same behavioral habits are matched as a third group, and users having the same period information in the third group are matched as a 3-1-th group. In this case, the period information indicates a date when the behavior pattern information is registered and an implementation period of the behavior pattern information. For example, a list of the matched groups may be provided to free users of this system.

Meanwhile, in the case of paid users of this system, for example, among users whose implementation rate of previously registered behavior pattern is greater than or equal to 60 % or 80 % or users having the implementation rate is greater than or equal to 80 % and a failure rate is less than or equal to 40 %, a group list may be provided by preferentially matching the users having the same period information as a 3-n-th group.

For example, referring to FIG. 8(a), when a user registers a behavior pattern and then requests user matching, the processor 230 may provide a group list display region 111 for displaying behavior pattern information of other users to each user in a group of users. Thereafter, a user may check behavior pattern information of other users in the matched group by performing a swipe touch in the group list display region 111. Subsequently, the user may check the behavior pattern information of other users, and then may be matched with each other through a cheer matching function 112 as illustrated in FIG. 8(b).

For example, the processor 230 may automatically establish a cheer relationship without mutual acceptance between users in a matched group. In this case, a direct message (DM) function between users is activated, and a report progress notification may be transferred between the users. In addition, a user may check a behavior pattern feed generated by another matched user and share additional behavioral habits, triggers, and solutions by using a comment function. Meanwhile, when mutually matched users achieve 100% of registered behavior patterns, reward neurons may be provided twice as much.

Accordingly, it is possible to increase a participation rate and motivate users to implement a solution for providing behavioral habit improvement of the users by strengthening solidarity between the users. In addition, the present disclosure has an effect of group therapy, which is one of cognitive behavior therapy (CBT), through social behaviors with other users, based on a service form of a social platform.

The processor 230 may recognize a previously received CBT-related qualification image based on artificial intelligence and approve an expert terminal (not illustrated), and when receiving an expert matching request from the user terminal 10, the processor 230 may match the approved expert terminal as an expert that coaches a user to improve behavior habits.

In addition, the processor 230 may provide a reward corresponding to the use information of coaching content to the expert terminal when use information of the user terminal 10 is checked for the coaching content provided by the expert terminal.

In additional embodiment, the processor 230 may match a user certified as an expert on a one-to-one basis upon receiving a user's expert matching request. Here, experts may be registered for each category through a CBT license or an equivalent certificate image authentication request. Users certified as experts may share their own content on the present system. Thereafter, as other users use the expert's content such as the number of views of content, corresponding compensation (points, coins, or so on) may be paid to the users certified as experts.

Hereinafter, descriptions of components performing the same function as each other among the configurations illustrated in FIGS. 1 to 8 are omitted.

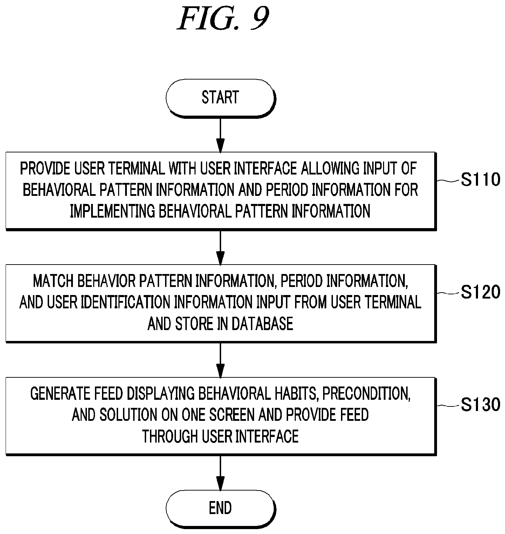

FIG. 9 is a flowchart illustrating a management method of providing behavioral habit improvement, according to an embodiment of the present disclosure.

Referring to FIG. 9, a method of providing behavioral habit improvement, according to an embodiment of the present disclosure, includes step S110 of providing the user terminal 10 with a user interface that allows an input of behavior pattern information including behavioral habits, triggers, and solutions, and period information for implementing the behavior pattern information, step S120 of matching the behavior pattern information, the period information, and user identification information, which are input from the user terminal 10, to each other and storing the matched information in the database 30, and step S130 of generating a behavior pattern feed that displays the behavioral habits, the triggers, and the solutions on one screen and providing the behavior pattern feed through a user interface.

In addition, the present disclosure further includes a step of receiving information on whether or not the behavior pattern information is implemented for each preset period from the user terminal 10, and a step of providing a visual image of an implementation rate based on the information on whether or not the behavior pattern information is implemented, through the user interface.

In this case, the behavioral habits correspond to a category that collectively refers to bad behavioral habits that need improvement, and include lack of sleep, an eating habit problem, a drinking problem, a non-smoking problem, a bad relationship, and lack of concentration.

In addition, the behavior pattern information is stored in the database 30 by being composed of a hierarchical structure including a plurality of triggers that cause at least one behavioral habit among categories and a plurality of solutions for preventing the behavioral habits by replacing at least one trigger.

In step S110, when a behavior pattern information registration request is received from the user terminal 10, a user interface is provided, wherein the user interface includes a first function for inputting at least one behavioral habit among categories, a second function for inputting at least one trigger that causes behavioral habits, a third function for inputting at least one solution that prevents the behavioral habits by replacing the trigger, and a fourth function for inputting period information including a period during which behavior pattern information is implemented, a start date, and a report date.

A method of providing a behavioral habit improvement service according to an embodiment of the present disclosure described above may also be implemented in the form of a recording medium including instructions executable by a computer, such as a program module executed by the computer. Computer-readable media may be any available media that may be accessed by a computer and include both volatile and nonvolatile media and removable and non-removable media. In addition, the computer-readable media may include all computer storage media. The computer storage media includes both volatile and nonvolatile media and removable and non-removable media implemented by any method or technology of storing information, such as a computer readable instruction, a data structure, a program module, and other data.

Although the method and system according to the present disclosure are described with reference to specific embodiments, some or all of their components or operations may be implemented by using a computer system having a general-purpose hardware architecture.

The above descriptions on the present disclosure are for illustration, and those skilled in the art to which the present disclosure pertains may understand that the descriptions may be easily modified into other specific forms without changing the technical idea or essential features of the present disclosure. Therefore, it should be understood that the embodiments described above are illustrative in all respects and not restrictive. For example, each component described as a single type may be implemented in a dispersed form, and likewise components described as distributed may be implemented in a combined form.

The scope of the present disclosure is indicated by the following claims rather than the above detailed description, and all changes or modifications derived from the meaning and scope of the claims and their equivalents should be interpreted as being included in the scope of the present disclosure.

## Claims

1. A management server for providing behavioral habit improvement, the management server comprising:
a communication module;
a memory configured to store a behavioral habit improvement service providing program; and
a processor configured to execute the program,
wherein the processor executes the program to provide a user terminal with a user interface that allows an input of behavior pattern information including behavioral habits, a trigger, and a solution, and period information for implementing the behavior pattern information, to match the behavior pattern information, the period information, and user identification information input from the user terminal, to store the matched information in a database, to generate a behavior pattern feed that displays the behavioral habits, the trigger, and the solution on one screen, and to provide the behavior pattern feed through the user interface.

2. The management server of claim 1, wherein
the processor receives information on whether or not the behavior pattern information is implemented for each preset period from the user terminal, and provides a visual image of an implementation rate based on the information on whether or not the behavior pattern information is implemented through the user interface.

3. The management server of claim 1, wherein
the behavioral habits correspond to a category that collectively refer to bad behavioral habits that need improvement and include lack of sleep, an eating habit problem, a drinking problem, a non-smoking problem, a bad relationship, and lack of concentration.

4. The management server of claim 3, wherein
the behavior pattern information is stored in the database by being composed of a hierarchical structure including a plurality of triggers that cause at least one behavioral habit in the category and a plurality of solutions for preventing the behavioral habits by replacing at least one trigger.

5. The management server of claim 3, wherein
the processor provides the user interface when receiving the behavior pattern information registration request from the user terminal, and
the user interface includes a first function for inputting at least one behavioral habit in the category, a second function for inputting at least one trigger that causes the behavioral habits, a third function for inputting at least one solution that prevents the behavioral habits by replacing the trigger, and a fourth function for inputting the period information including a period during which the behavior pattern information is implemented, a start date, and a report date.

6. The management server of claim 1, wherein
the processor matches, as one group, users having one or more pieces of the behavior pattern information matching each other which include the behavioral habits, the trigger, and the solution.

7. The management server of claim 1, wherein
the processor recognizes a previously received cognitive behavior therapy (CBT)-related qualification image based on artificial intelligence and approves an expert terminal, and matches the approved expert terminal as an expert that coaches a user to improve the behavior habits when receiving an expert matching request from the user terminal.

8. The management server of claim 7, wherein
the processor provides a reward corresponding to use information of coaching content to the expert terminal when the use information of the user terminal is checked for the coaching content provided by the expert terminal.

9. A method of providing behavioral habit improvement, the method comprising:
(a) providing a user terminal with a user interface that allows an input of behavior pattern information including behavioral habits, a trigger, and a solution, and period information for implementing the behavior pattern information;
(b) matching the behavior pattern information, the period information, and user identification information, which are input from the user terminal, to each other and storing the matched information in a database; and
(c) generating a behavior pattern feed that displays the behavioral habits, the trigger, and the solution on one screen and providing the behavior pattern feed through the user interface.

10. The method of claim 9, further comprising:
receiving information on whether or not the behavior pattern information is implemented for each preset period from the user terminal, and
providing a visual image of an implementation rate based on the information on whether or not the behavior pattern information is implemented, through the user interface.

11. The method of claim 9, wherein
the behavioral habits correspond to a category that collectively refer to bad behavioral habits that need improvement and include lack of sleep, an eating habit problem, a drinking problem, a non-smoking problem, a bad relationship, and lack of concentration.

12. The method of claim 11, wherein
the behavior pattern information is stored in the database by being composed of a hierarchical structure including a plurality of triggers that cause at least one behavioral habit in the category and a plurality of solutions for preventing the behavioral habits by replacing at least one trigger.

13. The method of claim 11, wherein, in (a),
the user interface is provided when receiving the behavior pattern information registration request from the user terminal, and
the user interface includes a first function for inputting at least one behavioral habit in the category, a second function for inputting at least one trigger that causes the behavioral habits, a third function for inputting at least one solution that prevents the behavioral habits by replacing the trigger, and a fourth function for inputting the period information including a period during which the behavior pattern information is implemented, a start date, and a report date.

14. The method of claim 9, wherein
users having one or more pieces of the behavior pattern information matching each other which include the behavioral habits, the trigger, and the solution are matched as one group.

15. The method of claim 9, wherein
a previously received cognitive behavior therapy (CBT)-related qualification image is recognized based on artificial intelligence to approve an expert terminal, and when an expert matching request is received from the user terminal, the approved expert terminal is matched as an expert that coaches a user to improve the behavior habits.

16. The method of claim 15, wherein
when use information of the user terminal is checked for coaching content provided by the expert terminal, a reward corresponding to the use information of the coaching content is provided to the expert terminal.
